# EUROPEAN PATENT APPLICATION

(11) **EP 4 578 487 A1**
(43) Date of publication of application: **02.07.2025**
(21) Application number: 23383394.6
(22) Date of filing: 28.12.2023
(51) Int. Cl.: A61N 1/04, A61N 1/36

(54) **A SYSTEM FOR BILATERAL AURICULAR NERVE STIMULATION OF THE VAGUS NERVE**

(71) Applicant: Xana Stim Sárl, 2822 Correaux (CH)
(72) Inventor: López Fernández, Miguel Ángel, Delemont (CH); Bermejo Velasco, Pedro Emilio, Bruselas (BE); García de Gurtubay, Iñaki, Pamplona (ES); Calvo Cuervo, David, Madrid (ES); Fernández Capdepont, José Enrique, Madrid (ES); Fuentes García, Carlos, Avilés (ES); Canteli Álvarez, Francisco, Gijón (ES); Fernández Fernández Vidal, Marina Carmen, Madrid (ES); Treceño García, Andrés Astur, Avilés (ES)
(74) Representative: Lehmann Novo, Maria Isabel

(57) **Abstract**

A bilateral auricular nerve stimulation system of the vagus nerve, which comprises two adjustable stimulator devices (4) in each ear respectively where each one comprises a support piece (5) with a curved temple (6); three or more cathodal stimulation electrodes simultaneously, with a first and second electrode (1, 2) that fit into the cymba and cavum respectively and a third electrode (3) that fits into the external auditory canal (EAC), where all electrodes are connected to one or more anodes (32), and have means of adjustment to their area of action; a photoplethysmography biosensor (PPG) (7) located at the free end of the curved temple (6), and; control means inside the support piece (5); where the system comprises a connection and synchronization cable between both stimulator devices.

## Description

### Technical field

The present invention corresponds to the technical field of medicine and specifically to auricular neurostimulation of the vagus nerve using electrodes.

### Background art

Currently, chronic pain, mental health and cardiological diseases, among others, are debilitating conditions affecting a significant portion of the population, with limited effective treatment options available. Existing stimulators are often limited in their capabilities and require customization by medical professionals to deliver effective therapeutic results efficiently.

The vagus nerve (VN) is the longest of the twelve cranial nerves. The VN connects the brainstem to the body, allowing the brain to receive control and monitor several body functions automatically. The VN is the main nerve of the parasympathetic division of the autonomic nervous system (ANS) that controls unconscious processes.

Because of this, multiple devices have come up with different ways of using vagus nerve (VN) stimulation to improve and treat such conditions.

The rationale of vagus nerve stimulation on the ear is based on anatomical studies demonstrating that certain parts of the ear area have afferent vagus nerve (VN) distribution thorough the auricular branch of the vagus nerve (ABVN). Cutaneous stimuli are transported via the auricular nerve to the jugular ganglion and from there with the VN into the medulla oblongata and to the nuclei tractus solitarii (NTS).

This nuclear region of the vagus nerve has multiple projections to many subcortical and cortical brain regions, which explains the long-distance physiological modulation of the vagus nerve stimulation. The desired therapeutic effect of aVNS depend on some critical points, such as:

### 1- The ability to depolarize the nucleus tractus solitarii (NTS):

The ultimate goal of stimulation is to deliver a dose of electrical charge sufficient to produce the desired physiological changes. The ABVN stimulation must guarantee the activation of the large, myelinated A-fibers, that generate nerve potentials on the NTS as the first step to spread the effect to higher levels.

The postsynaptic brainstem activity from the VN nuclei (yugular ganglion and NTS) can be recorded at the scalp as far field potential called vagus somatosensory evoked potential (VSEP). The amplitude of this evoked potential mainly reflects the number of electric potentials generated along the stimulated neural pathway as a representation of the amount of stimulated neural fibers and is only elicited when stimulating within the innervation area of the ABVN, while no VSEP will be recorded stimulating at other sites.

### 2- Anatomical target:

The specific ear region selected for stimulation is based on sparse and limited anatomical studies of human auricle dissection, which indicate several areas on the ear with different amounts of ABVN projections that can be used as stimulation targets. It is known that the cymba conchae of the ear is innervated exclusively by this branch, but other regions such as the cavum conchae, the tragus, and the posterior and inferior walls of the ear canal. of the external ear receive important afferent innervation by ABVN solely or shared with other auricular nerves.

### 3- The optimization of the current direction, shape of the stimulation pulse and stimulation protocol.

Anodic vs cathodic stimulation determines the stimulating current direction. In animals and humans, both anodic and cathodic pulses can evoke neural responses. So, anodic, cathodic, biphasic (anodic-cathodic) or other combinations could be used, differing on the effectiveness of evoking neural activation.

To date, most taVNS studies and devices employ monophasic stimulation (MS). Recently, because their electrical and physiological advantages other pulse shapes have been used as biphasic stimulation (BS), triphasic stimulation (TS) or other shape variations, such as DBP type stimulation, specifically designed to optimize the activation of neural fibres, lowering the stimulation threshold, and thus increasing the recruitment volume even more.

These new patterns have been successfully used with modulatory purposes over different diseases.

All these stimulation settings, including the stimulation time and the intensity at which it is performed, are those that determine the theoretical electrical dose to be administered. Most devices use a constant voltage approach (voltage-controlled stimulation) but, depending on the impedance, administered dose differ from the effectively received dose at the end of the taVNS session.

As an example of the state of the art, several existing devices on the market can be mentioned:
One of these devices, known as *GammaCore Sapphire*^{®}*,* is a handheld device that received United States Food and Drug Administration (FDA) clearance in 2015. Its purpose is to allow users to administer 2 minutes treatments for treating headaches and migraine stimulating one of the cervical branches of the vagus nerve in a non-invasive manner. For it, users are required to position the device against their neck with the intention of delivering impulses to the vagus nerve. Is rechargeable and is designed for long-term use. A doctor prescription is needed to activate the device.

A second medical device, known as Nemos^{®} by *tVNS*^{®}*,* has been specially developed to treat symptoms of sympathovagal imbalance that persist despite drug treatment such as epilepsy and migraine. It uses mild electrical stimulation through a special auricular electrode, placed on the left cymba conchae. This device requires over 4 hours of use to be efficient.

This stimulation, known as neuromodulation, is focused on the branch of the vagus nerve. The electrical impulses generated in this case are transmitted to the brain through specific nerve fibers, first in the brainstem and then in higher centers. This approach alleviates the symptoms of sympathovagal imbalance, and it is said that reduces seizures in cases of epilepsy. Patients can apply the stimulation independently, without requiring a medical professional.

Another device, *Parasym* ^{®}*,* is a neurostimulator that addresses health concerns related to the vagus nerve. The electrode is a clamp with two electrodes intended to be located in the left tragus. The treatment lasts at least 15 minutes per session.

Finally, the applicant himself is the owner of another device protected by the patent EP3693053. This device is specifically designed for stimulating the left auricular branch of the vagus nerve. In a recent study conducted by Gurtubay (2022), this device demonstrated significantly higher efficacy compared to conventional vagus nerve activators that stimulate only the cymba conchae. One key factor contributing to this device superior performance lies in its stimulation technique. By targeting two critical sites, namely the cymba and cavum, it optimizes the transmission of energy and harnesses the full potential of the vagus nerve. Currently, the device validated for the treatment of migraine and epilepsy using auricular vagus nerve stimulation (aVNS) delivers pulses only in the cymba. An essential feature of this device is its personalisation, as each earbud is customised for each individual user to maximise the contact between the electrodes and the skin.

However, these types of currently existing devices have certain drawbacks, as they have limited capabilities to activate the vagus nerve or require costly customization to offer effective therapeutic results.

### Summary of the invention

The system for bilateral auricular nerve stimulation of the vagus nerve covered by the present invention comprises two stimulator devices of the vagus nerve that are able to be adjusted to each user's ear respectively.

Each stimulator device comprises a support piece with a housing and a curved temple attached to said support piece by rotation means. This curved temple is able to be placed on the back of the ear to hold the stimulator device.

In addition, each stimulator device comprises three or more electrodes intended to deliver cathodic stimulation in the 3 sites. So that, a first and second electrode are adjusted in the cymba and cavum areas respectively to stimulate these areas, while a third electrode is adjusted in the external auditory canal (EAC) to stimulate this area.

Besides, all the electrodes are connected to one or more anodes, and they have adjustment means to their area of operation in the ear.

Each stimulator device also presents a photoplethysmography biosensor (PPG) located at the free end of the curved temple such that it is arranged in the posterior auricular area of the ear in contact with the skin, and control means connected to the electrodes and sensors that are located inside the support piece.

In addition, the system comprises a connection and synchronization cable between both stimulator devices.

With this system for bilateral auricular nerve stimulation of the vagus nerve a significant improvement over the state of the art is obtained.

This is because this system does not have the limitation of unilateral stimulation that current devices present, which target only one side of the nerve, which restricts the precision and effectiveness of the stimulation. Thus, to activate the vagus afferents, this system features two stimulator devices and each of them includes three main stimulation points in each ear. These points, cymba, cavum and EAC, are stimulated using the corresponding electrodes, effectively stimulating the branches of the vagus nerve. One or more anodes serve as a return path for the cathodic current.

Thus, this system introduces bilateral stimulation of the auricular nerve as a specific stimulation technique. This innovative approach involves electrical stimulation of the vagus nerve simultaneously in both ears. This bilateral stimulation technique offers new avenues to modulate vagal activity and potentially improve therapeutic outcomes.

In other way, this system achieves a comprehensive stimulation and is unique in its ability to cathodically stimulate three areas of great influx of the vagus nerve simultaneously. By targeting the cymba, cavum, and EAC regions, it provides a comprehensive stimulation that covers a wide range of vagal pathways. This comprehensive approach increases the chances of achieving desired physiological responses and therapeutic benefits.

Another advantage is that each stimulator device in the present system is specifically designed to fit the user's anatomy, ensuring optimal placement and contact with the intended nerves without the need for personalized customization. This design ensures superior contact unlike current standard auricular vagus nerve stimulators, resulting in significantly greater effectiveness.

This improves the accuracy and effectiveness of the device while reducing manufacturing time and costs. Additionally, by eliminating the need to restart the device and effectively managing voltage spikes, it offers greater convenience and a seamless experience for users.

In addition, the biocompatible and flexible electrodes used in each stimulator device maximize the contact area of the skin-electrodes and provide a comfortable and painless experience for the user, while still delivering stimulation at various points for maximum effectiveness.

Besides, the miniaturized earbuds and the headset-like design of each stimulator device is adjustable and non-invasive, making it comfortable and convenient for the user. Its ease of use and adaptability to individual needs increase its usability and accessibility. This user-friendly design promotes compliance and facilitates the integration of the device into clinical settings or at-home use.

An additional significant improvement is that each stimulator device incorporates a photoplethysmography sensor that enables to monitor cardiac functioning (HR/HRV) in real-time.

This is a crucial advantage as it guarantees the safety of the user and allows for the assessment of the effectiveness of stimulation on the autonomic nervous system. By studying the algorithmic analysis of cardiac variability, researchers and clinicians can gain valuable insights into the impact of stimulation on cardiac function and evaluate the therapeutic efficacy of the device.

Therefore, a very effective bilateral auricular nerve stimulation system of the vagus nerve is achieved that achieves maximum activation of the vagus nerve in a non-invasive way with the aim of developing a safe and efficient treatment for neurological medical pathologies.

### Brief description of the drawings

To complete the description and in order to provide for a better understanding of the invention, a set of drawings is provided. Said drawings form an integral part of the description and illustrate preferred embodiments of the invention, which should not be interpreted as restricting the scope of the invention, but just as an example of how the invention can be embodied.

The drawings comprise the following figures:
The Figure 1.- Shows a front and rear view respectively, of a stimulator device of a system for bilateral auricular nerve stimulation of the vagus nerve, according to a first embodiment of the present disclosure.
The Figure 2.- Shows a schematic view of the electronic circuit located within the housing of a stimulator device, according to a first embodiment of the present disclosure.
The Figure 3.- Shows a perspective view of both stimulator devices of a system for bilateral auricular nerve stimulation, according to a first embodiment of the present disclosure.
The Figure 4.- Shows a detailed view of the photoplethysmography sensor of a stimulator device, according to a first embodiment of the present disclosure.
The Figure 5.- Shows a perspective view of the first electrode and second electrode of a stimulator device, according to the first embodiment of the present disclosure.
The Figure 6.- Shows a detailed view of the first electrode, according to the first embodiment of the present disclosure.
The Figure 7.- Shows a detailed view of the second electrode, according to the first embodiment of the present disclosure.
The Figure 8.- Shows a detailed view of the third electrode, according to the first embodiment of the present disclosure.
The Figure 9.- Shows a perspective view of both stimulator devices of a system for bilateral auricular nerve stimulation, according to a second embodiment of the present disclosure.
The Figures 10.1 and 10.2.- Show a front view of a stimulator device and a detailed view of its first electrode respectively, according to a third embodiment of the present disclosure.
The Figure 11.- Shows a schematic view of the first electrode, according to a fourth embodiment of the present disclosure.
The Figures 12.1 and 12.2.- Show a front and rear perspective view respectively of a stimulator device, according to a fifth embodiment of the present disclosure.
The Figure 13.- Shows an internal schematic view of the stimulator device, according to the fifth embodiment of the present disclosure.
The Figure 14.- Shows a perspective view of both stimulator devices of a system for bilateral auricular nerve stimulation, according to a fifth embodiment of the present disclosure.
The Figures 15.1 and 15.2.- Show a first folded position and a second deployed position of the third electrode, according to a fifth embodiment of the present disclosure.
The Figure 16.- Shows a detail for the second electrode, according to the fifth embodiment of the present disclosure.
The Figure 17.- Shows a schematic diagram of the system for bilateral auricular nerve stimulation of the vagus nerve, according to the first embodiment of the present disclosure.
The Figure 18.- Shows a schematic diagram of the stimulator devices, according to the first embodiment of the present disclosure.
The Figure 19.- Shows a schematic image of an external accessory, according to a sixth embodiment of the present disclosure.
The Figure 20.- Shows a schematic image of external accessory placement, according to a sixth embodiment of the present disclosure.
The Figure 21.1, 21.2, 21.3.- Shows a schematic example of a way of using the external accessory placement for control the stimulator devices, according to a sixth embodiment of the present disclosure.

### Detailed description of the preferred embodiments

In view of the figures provided, it can be observed how in a first embodiment of the invention, the system for bilateral auricular nerve stimulation of the vagus nerve described here, operates over three sites of the auricular branch of the vagus nerve, those being: cymba, cavum, and external auditory canal (EAC). It comprises two stimulator devices (4) of the vagus nerve that are configured to be adjusted in each user's ear respectively.

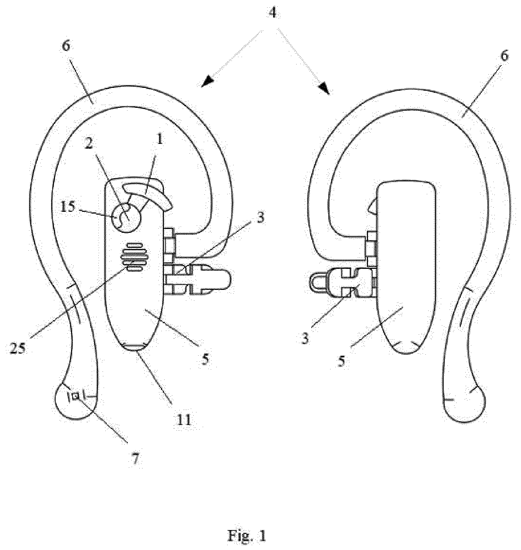

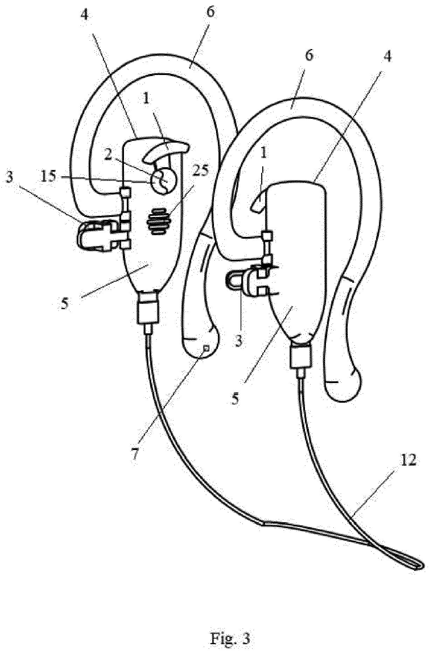

As show in figures 1 and 3, each stimulator device (4) comprises a support piece (5) with a housing and a curved temple (6) attached to said support piece (5) by rotation means. This curved temple (6) is configured to be placed on the back of the ear to hold the stimulator device (4) with a better grip. This temple (6) is made of an adjustable elastic material.

Also, each stimulator device (4) presents three or more electrodes intended to deliver cathodic stimulation simultaneously in the 2 ears.

As shown in figures 1 and 3, a first and second electrode (1, 2) are adjusted in the cymba and cavum areas respectively to stimulate these areas. Also, a third electrode (3) is adjusted to the external auditory canal (EAC) to stimulate this area.

All the electrodes are connected to one or more anodes, and they have adjustment means to their area of operation in the ear. In this first embodiment of the present disclosure each stimulator device (4) includes only one anode connected to the first, second and third electrodes (1, 2, 3).

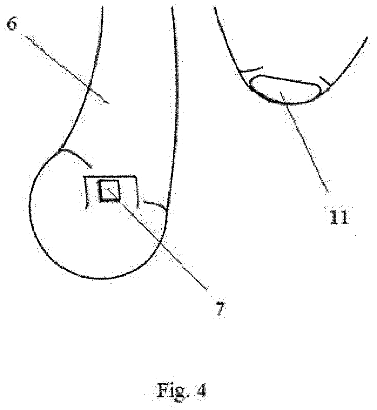

In this system, as shown in figures 1 and 4, each stimulator device (4) comprises a photoplethysmography biosensor (PPG) (7) located at the free end of the curved temple (6) such that it is arranged in the posterior auricular area of the ear in contact with the skin, as shown in detail in figure 4. The location of the photoplethysmography biosensor (PPG) located in the ear in the antitragus, or the posterior auricular being in contact with the carotid artery, allows monitoring the heart rate variability of users in real time, performing an important cardiological safety function.

This biosensor PPG (7) performs an estimation of the amount of hemoglobin and oxyhemoglobin circulating through the most superficial capillary vessels of the ear of the patient or user, once the device has been arranged on him or her. These data can be used to calculate the heart rate, the heart rate variability (HRV) and the oxygen saturation, among other biomedical variables and to detect the breathing phase of the user or patient.

The photoplethysmography or biosensor PPG (7) is able to detect a very low heart rate of the user and, in case this is detected, the device is configured to automatically stop the stimulation.

In addition, the measurements made by the sensor PPG (7) make it possible to know how much electrical charge needs to be applied to each user at any given time to achieve vagal activation. This allows personalizing the stimulation treatments reaching efficiency levels much higher than other existing devices known in the art.

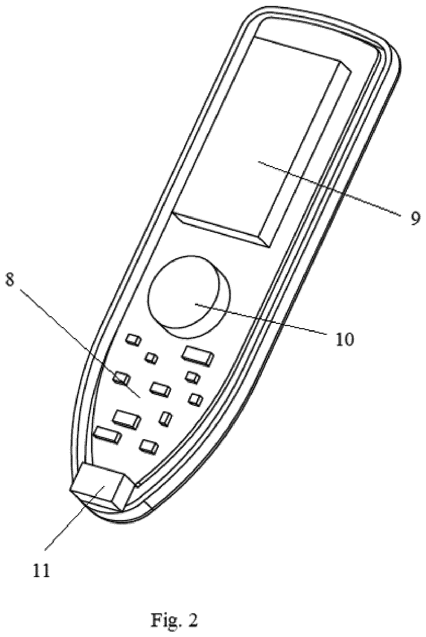

Each stimulator device (4) also comprises control means connected to the electrodes and sensors and located inside the support piece (5). In this first embodiment of the present disclosure, as show in figure 2, the control means comprise a printed circuit board (PCB) (8) with one or more microcontrollers and electronic components, a battery (9), a speaker (10) and connection means with the stimulator device (4), wherein the support piece (5) has a charging port (11) for the battery (9). The control means of both stimulator devices (4) are identical.

Besides, the system comprises connection and synchronization means (12) between both stimulator devices (4). As shown in Fig 3 for the first embodiment of the present disclosure, these connection and synchronization means (12) consist of a cable with connectors at the ends suitable for plugging into the charging port (11) at the end of each curved temple (6).

In a preferred embodiment, this charging port (11) is configured as a USB port and the two devices are connect by cable through the USB port, which also serves to recharge their batteries (9).

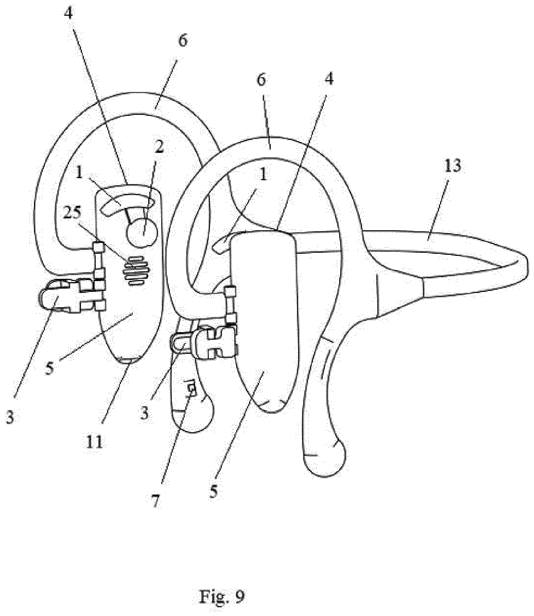

In a second embodiment of the present disclosure, as can be seen in Figure 9, the system comprises an adjustable and flexible headband (13) connected to both stimulator devices (4) at the respective curved temple (6) and the connection and synchronization means (12) are located inside this headband (13). The objective of this solution is to ensure maximum contact between the electrodes and the skin.

In the first embodiment of the present disclosure, as shown in Figures 1, 3, 5 and 6, the adjustment means of the first electrode (1) to the cymba area are formed by a curved longitudinal shape with a flexible and conductive surface that adapts to this area.

This first electrode (1) is configured as a working electrode on which a cathodic or stimulation is applied to maximize the activation of the auricular branch of the vagus nerve (ABVN). Its curved longitudinal shape like a banana features a similar shape to the anatomy of the cymba and possesses a flexible and conductive surface that guarantees the effectiveness of the electrical stimulation in the stimulation zone.

This first electrode (1) can rotate through a ball joint that is inserted into the housing of the support piece (5), allowing the adjustment of the first electrode (1).

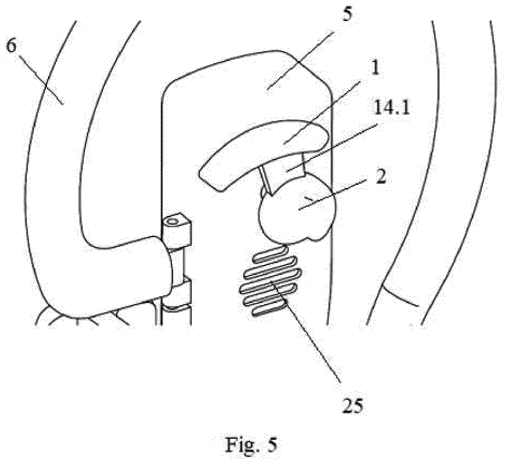

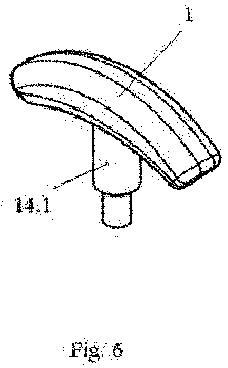

Figures 5 and 6 show a detailed view of the first electrode (1). This first electrode (1) comprises a support element (14.1) formed by a flexible yet rigid material, such that is able to be compressed into the cymba area ensuring constant contact with the skin and it is connected to the support piece (5) through a ball joint.

In this first embodiment of the present disclosure, as can be seen in Figures 1, 3, 5 and 7, the adjustment means of the second electrode (2) to the cavum area are formed by a rounded shape that adapts to this area and a ball joint for attachment to the support piece (5).

This second electrode (2) serves as a reference for applying the electrical voltage difference generated by cathodic stimulation. This electrode is rounded-shaped to maximize contact with the stimulation zone.

In addition, in this first embodiment of the present disclosure the first and second electrode (1, 2) are attached to the support piece by a same ball joint. This ball joint allows the first and second electrode (1, 2) to rotate in all directions and to adapt to different ears.

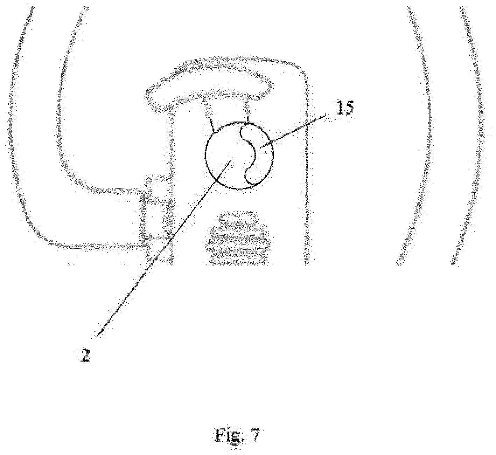

According to another aspect, as shown in Figure 7, the second electrode (2) has a raised crescent shaped area (15) that allows an improved contact of the second electrode (2) with the cavum area for the stimulation.

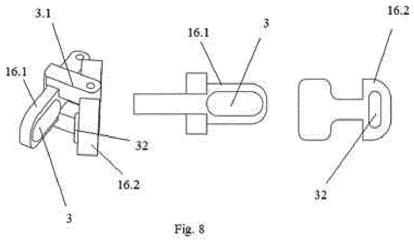

As can be seen in the figures 1, 3 and 8, in this first embodiment, the third electrode (3) is formed by a clamp (3.1) that can be fixed in the ear with a first elongated contact area (16.1) with cathodal stimulation, suitable to be in the external auditory canal (EAC), and a second contact area (16.2) where the anode is located.

Besides, in this first embodiment, the first area (16.1) with a cathode is positioned longitudinally and horizontally inside the external auditory canal (EAC), while the second area (16.2) makes contact on the outside of the ear tab and is in a vertical position.

The neurostimulator generates electric pulses by applying electrical voltage differences between the different electrodes. That's why system needs at least, one cathode and one anode. The neurostimulator connects different cathodes to one anode, according to the embodiments of the present first disclosure.

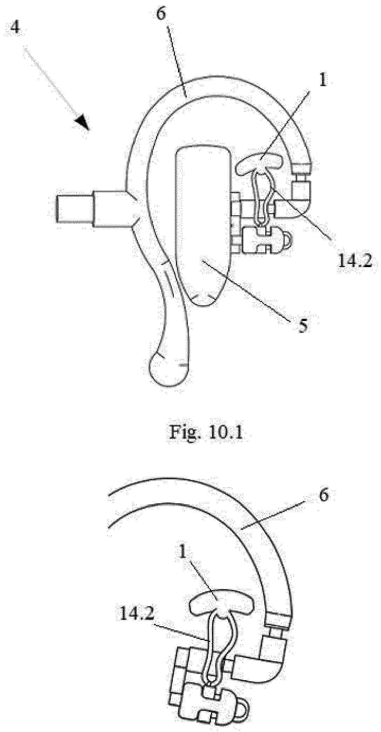

In a third embodiment of the present disclosure, as shown in Figures 10.1 and 10.2 the first electrode (1) comprises a support element (14.2) formed by a deformable elastic piece that allows a flexible and tight fit of the first electrode (1) in the cymba area and it is connected to the clamp of the third electrode (3).

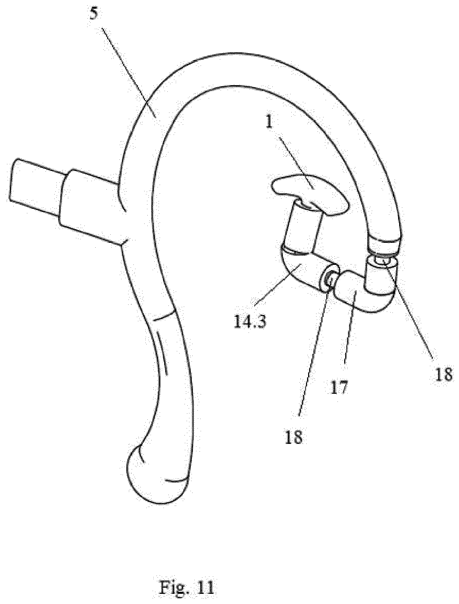

In another configuration, in a fourth embodiment of the present disclosure, as shown in Figure 11, the first electrode (1) maintains its original design, while the support undergoes changes. The support now comprises of two movable rotaries that enable the electrode to move in four different axes: In and out of the ear and back and forward. It comprises a support element (14.3) with an intermediate element (17), wherein the intermediate element (17) is connected by ball joints (18) to the support element (14.3) and to the curved temple (6).

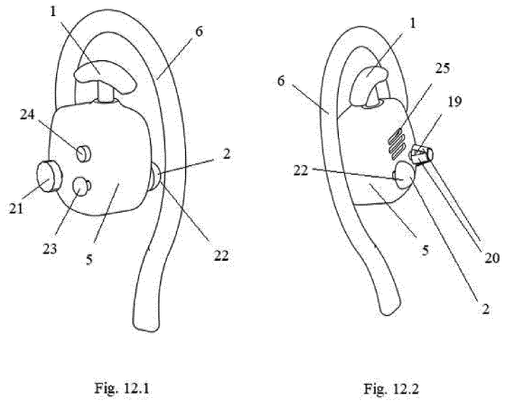

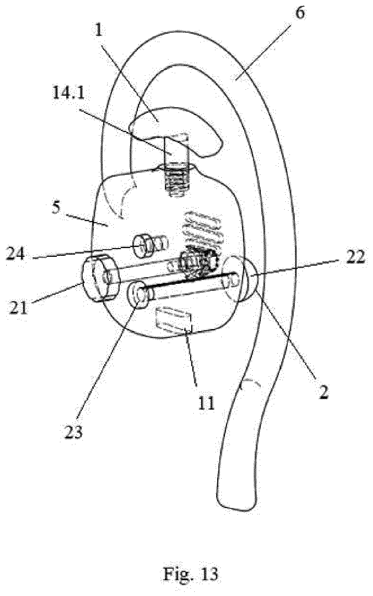

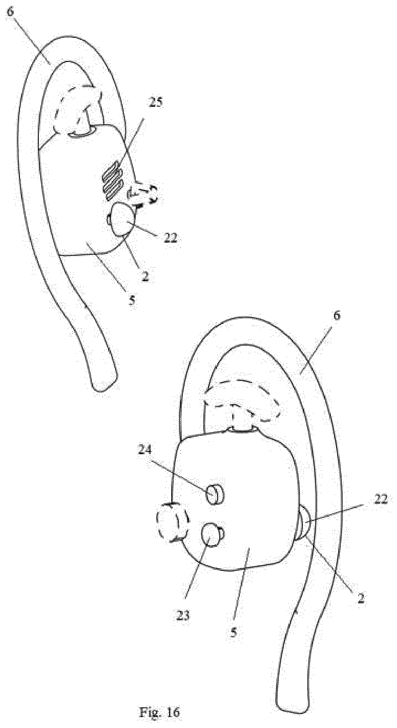

A fifth embodiment of the present disclosure is depicted in Figures 12.1, 12.2 to 16. In this case, the third electrode (3) comprises a central element (19) of cylindrical shape with inclined walls suitable to be placed in the EAC. At the end of this central element (19) there are hingedly connected a plurality of inclined laminar pieces (20). At least two of the laminar pieces (20) are a cathode and an anode respectively.

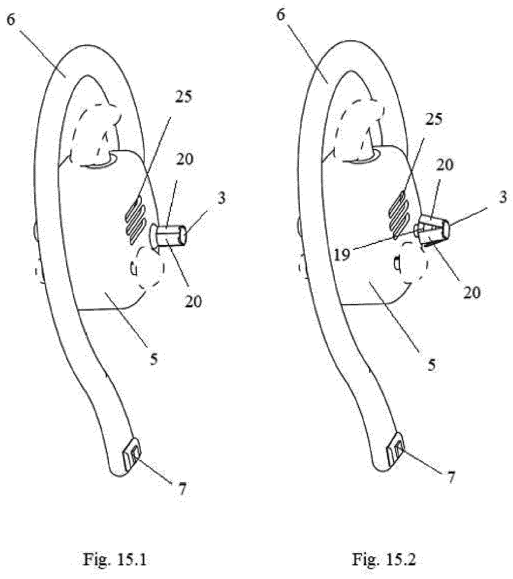

Furthermore, these laminar pieces (20) have a first folded position over the central element (19), as shown in Figure 15.1, and a second deployed position to connect to the EAC, as can be seen in Figure 15.2.

In this case, this configuration of the third electrode (3) allows it to be opened and folded while remaining attached to the central element (19) to facilitate its insertion into the EAC cavity.

Both the cathode and the anode are placed on two of the laminar pieces (20) and the number of laminar pieces (20) is variable. The figures 12.1, 14, 15.1 and 15.2 show an example of an embodiment with four laminar pieces (20), where one of them acts as the cathode, one as the anode while the remaining two are simple clamping pieces.

As indicated, these laminar pieces (20) are foldable, i.e., they can be folded remaining attached over the central element (19) to facilitate insertion into the EAC channel and once inserted, acting the user on an actuator (21), they can be opened in the form of petals, to achieve a better connection with the walls of the EAC channel. The degree of opening of the laminar pieces (20) is adjustable by means of the actuator (21).

In this fifth embodiment of the present disclosure the first electrode (1) is the same as that of the first embodiment (shown in Figure 6). The extension of the support element (14.1) is inserted into a hole provided in the support piece (5) where it is fixed and connected to an elastic spring that enables its vertical displacement, thus improving the fit over the ear.

In this fifth embodiment the second electrode (2) has a different configuration. As shown in figures 12.1, 12.2, 13, 14 and 16, The second electrode (2) is positioned on a hemispherical part (22) which in turn is connected by a shaft to an actuating button (23). The operation of the actuating button (23) allows a first retracted position and a second extended position inside the cavum area.

This model is fully adjustable to the anatomy of the user's ear. Pressing the actuating button (23) moves the shaft of the second electrode (2). It should be pushed until the hemispherical part (22) touches the cavum wall. Once the optimal set point is reached, the electrode locks its movement by the user pressing a locking button (24). This locking button (24) is used to lock the second electrode (2) in a certain position and also to unlock it when desired.

With this design, not only is efficiency ensured by maximizing the contact between the second electrode (2) and the skin, but also provides substantial cost savings because it does not need to be customized. Thus, industries obtain optimized processes, reduced downtime and higher overall productivity. This innovative technology ensures better allocation of resources, reducing waste and maximizing production, resulting in increased profitability and long-term sustainability.

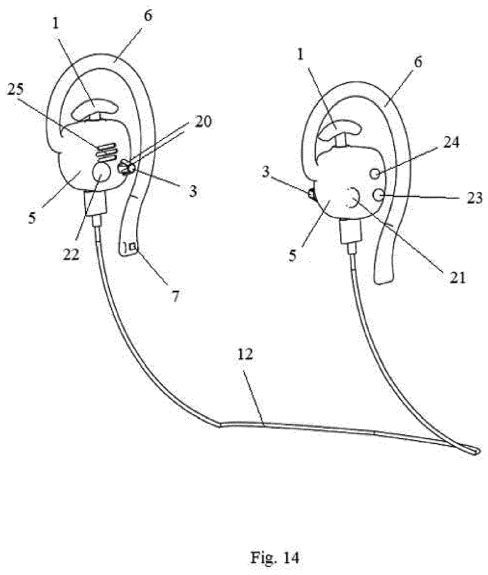

In this fifth embodiment, the system also comprises a connection and synchronization cable (12) between both stimulator devices (4) as shown in Fig 14 being the functionality configured in the same way as described for the first embodiment. The two devices are connected by cable through the lower connection port. Although not shown in the figures, both stimulator sets can also be connected by means of a headband as described for the first embodiment.

In all embodiments the electrodes are typically manufactured using graphene, biocompatible metals such as titanium, nickel titanium (nitinol), platinum, platinum-iridium, non-toxic metals such as gold, biocompatible conductive inks for 3D printing or flexible biocompatible conductive polymers that adapt to the anatomy of the stimulation area, thus providing good comfort and a perfect fit to the patient's ear.

The housing of the support piece (5) is formed by biocompatible and rigid material. This support piece (5) is used as a support for the first, second and third electrodes (1, 2, 3) respectively in order to ensure that the positioning of these electrodes is adequate to maximize stimulation. The advantage of this housing in each stimulator device (4) of the invention is that it presents a standard configuration, which can be used for any patient, without the need for customization.

In the first embodiment of the present disclosure, this housing of the support piece (5) has grilles (25), at shown in figures 1, 3, 5, 7, that perform a double function of ventilation and also serve as a speaker. The stimulator device (4) is configured to transmit simple basic messages to the user during its operation (Ex: Low battery, connection lost, etc.).

As already indicated, the system comprises control means in each stimulator device (4) with a PCB (8), allowing it to develop the following functionalities, that are indicated below.
- Generation of stimulation patterns with duration, intensity, frequency of bursts and variable pulses, number of pulses per burst, pulse widths and pulse delays, among others, by applying electrical voltage differences between the different electrodes.
- Control of electrical load applied in each stimulation and daily accumulated load.
- Data exchange with external devices through wireless connections.
- Personalized treatments for each stimulator device (4), allowing different stimulation intensities on each side at the same time if necessary.
- Connection with other electronic devices (e.g a smartphone or a tablet) using wireless technology for remote control of the stimulator device (4). As both stimulation sets are connected by cable (12), it is only necessary to pair one of the stimulation devices with the smartphone.

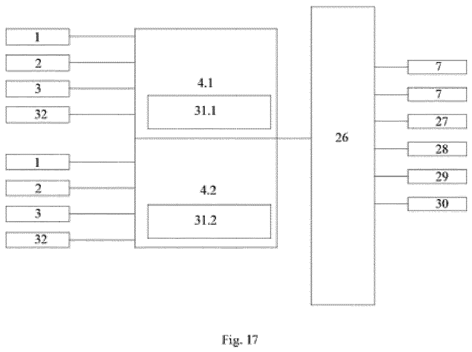

Figure 17 shows a schematic diagram of the system for bilateral auricular nerve stimulation of the vagus nerve, for the first embodiment of the present disclosure.

The system comprises of a stimulation device (4) for each ear plus some additional peripherals and contains two different subsystems: right (4.1) & left (4.2), with identical characteristics, where each of them corresponds to one of the stimulator devices.

Those two subsystems as well as the peripherals are managed by the Logic & Control (26) unit of the PCB.

Summarizing, the system consists of:
- Electrodes (1,2,3) connected to one or more anodes (32)
- Bilateral Auricular Neurovagal stimulator device (4): Right (4.1) and Left (4.2)
- Logic and control (26)
- Feedback: Photoplethysmography (7) (Right and Left)
- Peripherals: Bluetooth (27), Wi-Fi (28), UI (User interface) (29), Audio (30)

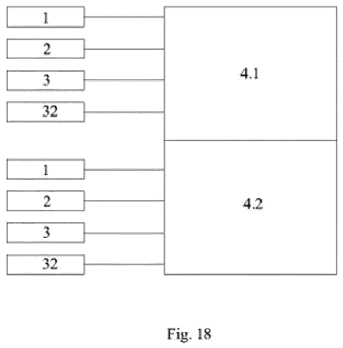

Figures 17 and 18 show that each of both subsystems (4.1, 4.2) include four electrodes as well as the required electronic to be able to transform the specific pulse signals generated by the Logic&Control unit (26) in an appropriated pulse signal with a defined voltage and a limited current. Due to the required voltage is in a range which cannot be directly supplied by the Logic&Control unit (26), each stimulator device (4) includes an adapt section (31.1, 31.2) with the needed electronic for the signal adaptation.

For this section at least three different pulse signals per side can be defined and managed from the Logic&Control unit (26). Those six pulse signals are independent allowing different pulse configuration for each one (different characteristics like frequency, amplitude, wave shape, ...)

Besides of it, current/voltage is monitored as feedback from the electrode to the Logic & Control unit (26) to ensure the proper behavior of the pulse generator section in real time.

The logic & control unit (26) consist of a microcontroller or a group of microcontrollers with all the associated electronic required to work like power supply, oscillator, etc.

This logic & control unit (26) is responsible of managing and controlling all the other modules. This includes, among others, the following functions:
- All the pulse signals are generated by this logic & control unit (26) according to the parameters defined using the Bluetooth (27), Wi-Fi (28) or any other User Interface (29).
- Process all the collected data to be sent to the cloud (PPG data)
- Control the peripheral modules like Audio, LEDs, digital inputs.
- Collect the feedback of the pulse signal generated.

- All the software is included in this Logic & Control unit (26).

To monitor the different variables identified during the concept phase, the Feedback Module collects information from several sensors to be received directly by the Logic & Control unit (26). In the embodiments described, the inputs are at least the PPG (7) Right and left, allowing to receive information derived from the blood pressure changes. As this signal is received from two different sources, processing can be implemented in the Control & Logic units (26) to improve this data point quality.

A sixth embodiment of this invention is possible in which the operation of the bilateral vagus nerve stimulation system is combined with an external accessory that allows the activation of the stimulator devices only when the subject performs certain movements.

The release of the pulse would be optional according to the data obtained from an accelerometer located in a limb (arm, leg) so that when a patient with paralysis in this limb moves it during a rehabilitation task, the stimulation is activated. It has been demonstrated that, in these patients with a paralyzed limb (for example, after suffering a stroke), if the stimulation is produced only during the execution of the movement, the recovery is greater.

Thus, when the accelerometer detects that the subject has moved the limb, the stimulation is triggered. The scientific basis for this mechanism has to do with the ability of the vagus nerve to increase the plasticity of the nervous system.

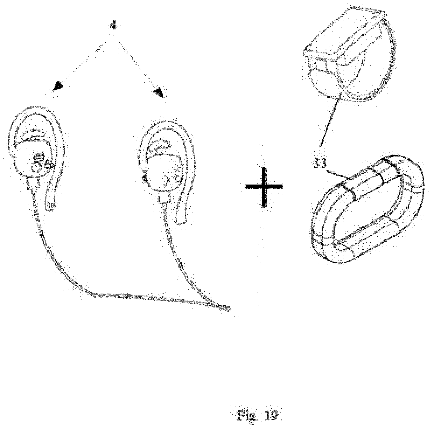

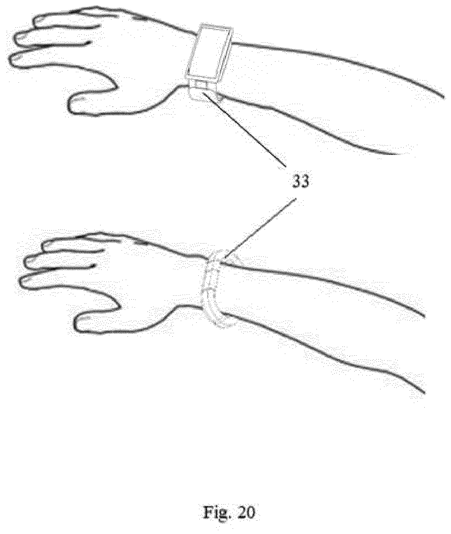

Some possible embodiments of this external device are shown in figures 19, 20 and 21.1 to 21.3.

The accessory consists of a bracelet or an adjustable velcro strap (33) that incorporates simple electronics to control it and connect the device via Bluetooth with the stimulator devices (4) and also an accelerometer that detects movements by the user. Figure 19 shows both alternatives: an example of an adjustable Velcro strap is shown at the top and an alternative bracelet configuration at the bottom.

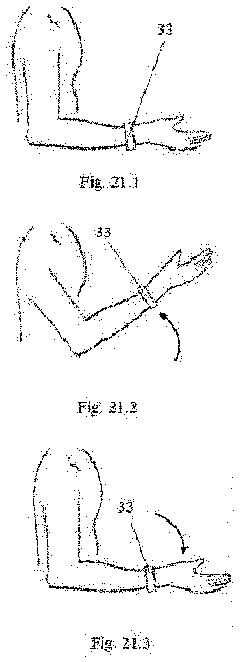

A graphic example of how the system works is shown in Figures 21.1-21.3. Figure 21.1 shows how when the subject is at rest, the stimulator devices are turned off and do not provide any type of stimulation.

When the user begins to make certain movements based on his needs, as depicted in Figure 21.2, the accelerometer of the bracelet or Velcro strap (33) detects the movement and orders the stimulator devices to release the stimulation.

Upon returning to the resting position as can be seen in Figure 21.3, the stimulation stops again until the next movement.

These external accessories (bracelets o Velcro straps) can be used in the same way described above by placing it on the wrist, forearm and leg, depending on the area to work on.

The device described in the patent has a wide range of potential applications, primarily in the field of neurostimulation and vagal nerve modulation. Some of the possible applications include:
Neurological Disorders: The device may be used in the treatment of various neurological disorders such as epilepsy, migraine, cluster headaches, and chronic pain. By stimulating the vagus nerve in multiple areas, it has the potential to modulate neural circuits and reduce the frequency and severity of symptoms associated with these conditions.
Mood Disorders: Vagus nerve stimulation has shown promising results in treating mood disorders such as depression and anxiety. The device's ability to stimulate multiple areas of vagus nerve influx can provide a more targeted approach to modulating neural pathways involved in mood regulation.
Cardiology: Disorders of autonomic functioning, such as heart failure, can benefit from vagal nerve stimulation. By monitoring cardiac variability in real-time, the device can help assess the effectiveness of the stimulation in regulating autonomic function and potentially improve symptoms associated with autonomic dysfunction.
Cognitive Enhancement: Vagus nerve stimulation has been explored as a potential treatment for cognitive impairments and neurodegenerative disorders. The device's comprehensive stimulation of the vagus nerve can potentially enhance cognitive functions such as memory, attention, and executive function.
Rehabilitation: In the field of physical rehabilitation, vagal nerve stimulation has been studied for its potential to enhance motor recovery after stroke or spinal cord injury. The device could be utilized in conjunction with rehabilitation protocols to facilitate neural plasticity and improve functional outcomes.
Research and Development: The device's ability to stimulate the vagus nerve in multiple areas and monitor cardiac variability opens new avenues for research and development. Scientists and clinicians can utilize the device to study the effects of vagal nerve modulation on various physiological processes, enabling a better understanding of the intricate connections between the autonomic nervous system and various health conditions.

It is important to realize that these applications are hypothetical and would require further research and clinical trials to determine the device's efficacy and safety in each specific use case.

## Claims

1. A system for bilateral stimulation of three sites of the auricular branch of the vagus nerve, those being cymba, cavum, and external auditory canal (EAC), comprising two stimulator devices (4) of the vagus nerve configured to be adjusted in each user's ear respectively, wherein each stimulator device (4) comprises
- a support piece (5) with a housing and a curved temple (6) attached to said support piece (5) by rotation means, where the curved temple (6) is configured to be placed on the back of the ear to hold the stimulator device (4);
- three or more electrodes intended to deliver cathodic stimulation simultaneously, wherein a first and second electrode (1, 2) are adjusted in the cymba and cavum areas respectively to stimulate these areas, while a third electrode (3) is adjusted in the external auditory canal (EAC) to stimulate this area, wherein all the electrodes are connected to one or more anodes (32), and they have adjustment means to their area of operation in the ear;
- a photoplethysmography biosensor (PPG) (7) located at the free end of the curved temple (6) such that when the device is in the in-use position, it is positioned in the posterior auricular area of the ear in contact with the skin close to the carotid artery, and;
- control means connected to the electrodes and sensors that are located inside the support piece (5);
wherein the system comprises connection and synchronization means (12) between both stimulator devices (4).

2. The system of claim 1, where the third electrode (3) is formed by a clamp (3.1) that can be fixed in the ear with a first elongated contact area (16.1) with cathodic stimulation, suitable to be located in the EAC, and a second contact area (16.2) where the anode is located.

3. The system according to claim 2, the first area (16.1) with a cathode is positioned longitudinally and horizontally inside the external auditory canal (EAC), while the second area (16.2) makes contact on the outside of the ear tab and is in a vertical position.

4. The system according to any of claims 1-3, wherein the first electrode (1) has a curved longitudinal shape similar to the anatomy of the cymba and comprises a flexible and conductive surface.

5. The system of claim 4, wherein the first electrode (1) comprises a support element (14.1) formed by a material such that is able to be compressed into the cymba area and it is connected to the support piece (5) through a ball joint.

6. The system according to any of claims 1-4, wherein the first electrode (1) comprises a support element (14.2) formed by a deformable elastic piece that allows a flexible and tight fit of the first electrode (1) in the cymba area and it is connected to the clamp (3.1) of the third electrode (3).

7. The system of claim 4, wherein the first electrode (1) comprises a support element (14.3) with an intermediate element (17), wherein the intermediate element (17) is connected by ball joints (18) to the support element (14.3) and to the curved temple (6).

8. The system of claim 1, wherein the third electrode (3) comprises a central element (19) of cylindrical shape suitable to be placed in the EAC, wherein at the end of this central element (19) there are hingedly connected a plurality of inclined laminar pieces (20), and wherein at least two of the laminar pieces (20) are a cathode and an anode respectively and wherein these laminar pieces (20) can be folded in a first position folded over the central element (19) and a second deployed position to connect with the EAC.

9. The system according to any of claims 1-8, wherein the second electrode (2) has a rounded shape that adapts to the cavum area and a ball joint for attachment to the support piece (5).

10. The system according to claims 5 and 9, wherein the first and second electrodes (1, 2) are attached to the support piece (5) by a same ball joint.

11. The system according to any of claims 9 or 10, wherein the second electrode (2) has a raised crescent shaped area (15) that allows an improved contact of the second electrode (2) with the cavum area.

12. The system according to any of claims 1-8, wherein the adjustment means of the second electrode (2) is positioned on a hemispherical part (22) which in turn is connected by a shaft to an actuating button (23); the operation of the actuating button (23) allows a first retracted position and a second extended position inside the cavum area.

13. The system according to any of claims 1-12, wherein the control means comprise a printed circuit board (PCB) (8) with one or more microcontrollers and electronic components, a battery (9), a speaker (10) and connection means with an electronic device, wherein the support piece (5) has a charging port (11) for the battery (9).

14. The system according to any of claims 1-13, comprising an adjustable and flexible headband (13) connected to both stimulator devices (4) on the respective curved temple and the connection and synchronization means (12) are located inside this headband (13).

15. The system according to any of claims 1-14, wherein the housing of the support piece (5) has grilles (25).
